# EUROPEAN PATENT SPECIFICATION

(11) **EP 3 300 651 B1**
(45) Date of publication and mention of the grant of the patent: **29.01.2020**
(21) Application number: 17194306.1
(22) Date of filing: 02.10.2017
(51) Int. Cl.: A61B 1/00, A61B 1/06, A61B 1/07

(54) **LOW PROFILE ENDOSCOPE**
ENDOSKOP MIT NIEDRIGEM PROFIL
ENDOSCOPE À PROFIL BAS

(30) Priority: 30.09.2016 US 201662402145 P
(43) Date of publication of application: 04.04.2018
(73) Proprietor: Cook Medical Technologies LLC, Bloomington, IN 47404 (US)
(72) Inventor: MELSHEIMER, Jeffry S., Springville, IN 47462 (US)
(74) Representative: Mathys & Squire

(56) References cited:
- EP-A2- 0 276 139
- WO-A1-2015/198743
- JP-A- 2008 228 953
- US-A1- 2008 281 158

## Description

### BACKGROUND

The present disclosure relates generally to image devices for medical use, and particularly, to endoscopes for insertion within a body vessel or cavity having a low profile for additional use as a guidewire.

Endoscopes and other like imaging systems have been developed to display an image on a video monitor for a physician or operator to investigate symptoms of vessel or cavity, confirm diagnosis of condition, and provide treatment of condition. Endoscopes typically include rigid or flexible tubular housings for supporting objectives of one or more glass lenses, a light for illumination of the object under inspection, and an image sensor proximate the objective that interfaces with the objective and converts the incoming light from the objective into digital signals. Digital signals are coupled directly to a video processor or indirectly to a remote video processor that the operator uses to observe the object under inspection. Light is directed outside the housing typically through fiber optics that are coupled to a light source outside of the body.

Some areas of the body, such as bile duct, ureter, or kidneys, require minimally sized endoscopes. For such procedures, a guidewire is inserted into the vessel or cavity under fluoroscopy initially, and the endoscope is inserted along the guidewire. The guidewire remains in the body to guide other medical devices such as catheters, stent deployment devices, lithotripsy, laser or basket devices for additional treatment procedures. Due to small diameters of some of these small regions, multiple devices side by side may make it difficult to navigate the devices, making it more painful and providing more discomfort for the patient or providing additional trauma to the tissue.

Document EP 0 276 139 A2 discloses an endoscope insertion section with an electrical connector with ring electrodes and an optical connector with a central circular window for illumination light.

### SUMMARY

The invention is defined in the appended claims. According to the invention, an endoscope is provided. The endoscope includes an elongated body having an outer liner that defines a lumen disposed about a longitudinal axis. The body has a proximal end and a distal tip. The outer liner has at least a portion of a light transmittable jacket disposed about the distal tip. An imaging system includes a lens and an image device disposed within the lumen at the distal tip. A plurality of image conductors extend within the lumen between a distal end and proximal end, with the distal end of the image conductors coupled to the image device. A plurality of light conductors is arranged at the distal tip. The light conductors have a distal end and a proximal end and extend within the lumen therebetween. The distal end of each of the light conductors is in optical communication with the light transmittable jacket at a location proximal to the image device. A serial electrical connector is formed along the proximal end of the body. The serial electrical connector includes a plurality of ring conductors spaced from one another and an annular window segment axially disposed relative to the ring conductors. Each of the ring conductors is associated with and in electrical communication with the proximal end of one of the image conductors. The annular window segment is in optical communication with the proximal end of the light conductors.

In another example, an elongated body of an endoscope has an outer liner defining a longitudinal lumen about a longitudinal axis. The body has a proximal end and a distal tip. A lens and an image device are disposed within the lumen at the distal tip. A plurality of image conductors extends within the lumen from the image device. A distal refractor is disposed within the lumen proximal to the image device and the lens. A proximal refractor is disposed proximal to the distal refractor. A plurality of light conductors is coupled between the distal and proximal refractors. An outer liner has jackets circumferentially disposed about the distal refractor and the proximal refractor, with the jackets comprising a light transmittable material.

In another example, a vision system including an endoscope and an electrical-light connector is provided. An elongated body of the endoscope has an outer liner defining a longitudinal lumen about a longitudinal axis, with the body having a proximal end and a distal tip. A lens and an image device are disposed within the lumen at the distal tip. A plurality of image conductors extends within the lumen from the image device. A plurality of light conductors is arranged at the distal tip, having a distal end and a proximal end. The distal end of each of the light conductors is in optical communication with a light transmittable jacket segment disposed at the distal tip. A serial connector of the endoscope is formed along the proximal end of the body. The serial connector includes an annular window segment that is in optical communication with the proximal end of each of the light conductors. The electrical-light connector is removably attachable to the serial connector of the endoscope. The electrical-light connector has a surface that defines a channel to receive the serial connector. The surface includes a light window segment in optical communication with a light source housed in the electrical-light connector. When the serial connector is received by the channel of the electrical-light connector, the annular window segment and the light window segment are in alignment to allow for optical communication from the light source to the proximal end of the light conductors.

Other systems, methods, features and advantages of the invention will be, or will become, apparent to one with skill in the art upon examination of the following figures and detailed description.

### BRIEF DESCRIPTION OF THE DRAWINGS

The invention can be better understood with reference to the following drawings and description. The components in the figures are not necessarily to scale, emphasis instead being placed upon illustrating the principles of the invention. Moreover, in the figures, like referenced numerals designate corresponding parts throughout the different views.
FIG. 1 is a side view of one example of an endoscope.
FIG. 2 depicts another device sliding over a proximal end of the endoscope of FIG. 1.
FIG. 3 is a longitudinal cross-sectional view of a distal tip of the endoscope of FIG. 1.
FIG. 4 is an axial cross-sectional view taken along lines 4-4 in FIG. 3.
FIGS. 5A-5B are side views of examples of distal and proximal refractors.
FIG. 6 is an end view of the distal refractor in FIG. 5A.
FIG. 7 depicts a subassembly of an example of an illumination system disposed within an endoscope.
FIG. 8 is a longitudinal cross-sectional view of a proximal region of the endoscope of FIG. 1.
FIG. 9 is a longitudinal cross-sectional view of a distal tip of another example of an endoscope.
FIG. 10 is an axial cross-sectional view taken along lines 10-10 in FIG. 9.
FIG. 11 is a longitudinal cross-sectional view of a proximal region of the endoscope of FIG. 9.
FIGS. 12-13 depict an electrical-light connector for coupling to the endoscope moving between a closed position and an open position.
FIG. 14 depicts the closing of the electrical connector after a steerable catheter has been slid over the endoscope.
FIG. 15 depicts a distal tip of a treatment catheter sliding over the endoscope.
FIG. 16 depicts the manufacturing of a serial connector of the endoscope.

### DETAILED DESCRIPTION OF THE PREFERRED EMBODIMENTS

Endoscopes with a low profile for body vessels and cavities are described herein. The endoscopes may be used as a medical guidewire over which other medical devices may be placed and introduced into body vessels and cavities of a patient, in addition to its use as an image system for navigation and diagnosis. Medical devices may be onloaded or offloaded from the proximal end and/or the distal end of the endoscope. In order to quickly transition the onloading and offloading process, an electrical-light connector is described herein to be coupled and decoupled from the proximal end of the endoscope. To this end, devices for steering the endoscope and devices for treatment of condition may be more easily exchanged from the endoscope. This configuration can also allow the removal of additional parallel structures such as conventional guidewires that increase the profile created by the medical devices. The endoscope and other devices make navigation easier, cause less pain to the patient, and cause less trauma to the body tissues, especially relatively small diameter vessels, such as but not limited to, urethras and ureters.

In the present application, the term "distal" when referring to a delivery device refers to a direction that is farthest away from an operator using a delivery device, while the term "proximal" refers to a direction that is generally closest to the operator using the delivery device. The distal and proximal ends of a delivery device may also be referred to as an introduction end of the delivery device and an operator end of the delivery device, respectively. The term "operator end" of the delivery device is that portion of the device that is intended to remain outside of a patient during a procedure. The term "introduction end" of the delivery device, which is opposite to the operator end, is that portion of the device that is intended to be inserted within a patient during a procedure.

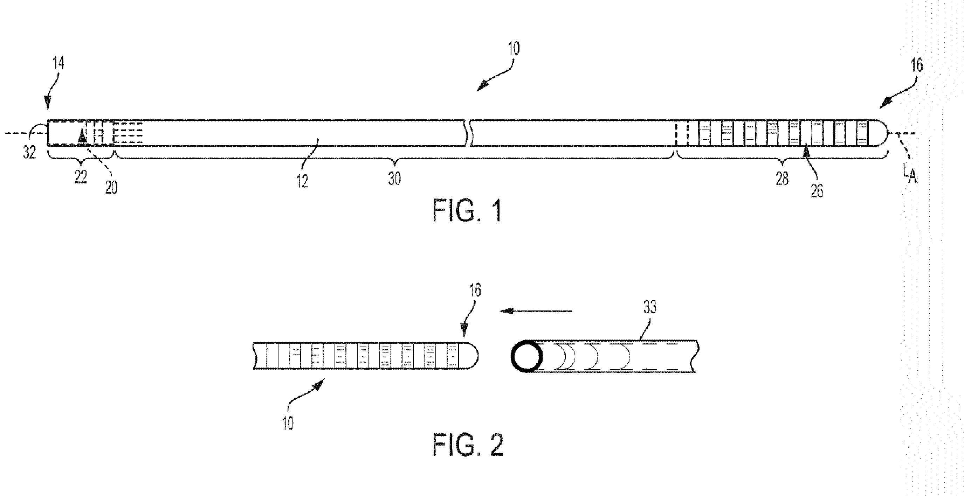

**FIG. 1** depicts an example of an endoscope 10. The endoscope 10 comprises a flexible elongated endoscope body 12 extending about a longitudinal axis LA between an introduction, distal end 14 for insertion into the body vessel or lumen and an operator, proximal end 16 for remaining outside the body of the patient. An imaging system 20 may be arranged at a distal tip 22 of the endoscope body 12 along the distal end 14. The imaging system 20 includes a lens 40 and an image device 50 for capturing an image, and an illumination system 60 for illuminating an object outside a distal face 32. A serial connector 26 may be arranged along a proximal region 28 of the proximal end 16. The endoscope 10 includes an intermediate region 30 between the distal tip 22 and the proximal region 28. The axial end of the distal tip 22 may be referred to as the distal face 32.

As depicted, the endoscope body 12 may be low profile. One of the benefits of a low profile configuration is depicted in **FIG. 2****.** Here, the endoscope 10 is shown having additional uses as a guidewire for other devices, where the other device (for example, a steering catheter 33) can be slidably inserted over the proximal end 16 or the distal end 14 of the endoscope body 12. In some examples, the endoscope body 12 has a uniform cross-section along the entire body. Alternatively, the intermediate region 30 may have a reduced cross-sectional area relative to the distal tip 22 and the proximal region 28. In yet another alternative embodiment, the cross-sectional area of the distal tip 22 may be at least as large as the proximal region 28, and in some examples, may be greater than the cross-sectional areas of the intermediate region 30 and the proximal region 28.

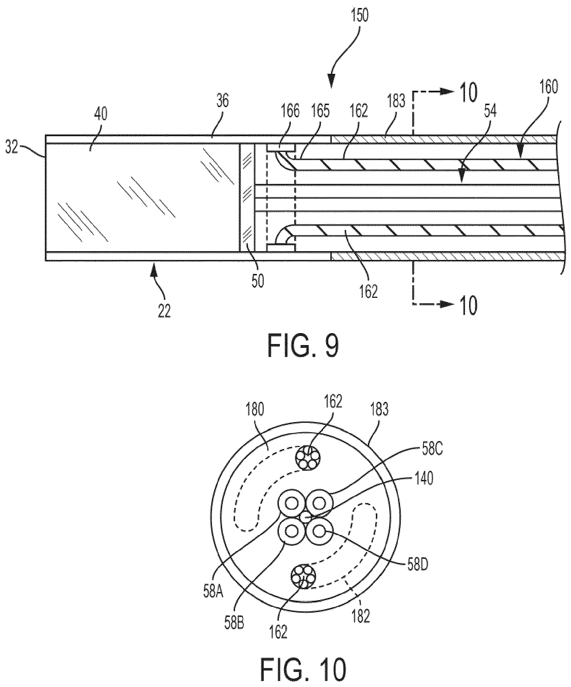

The endoscope body 12 may include an outer liner 34 defining generally a body lumen 35 along the longitudinal axis LA. For example, the distal tip 22 may be surrounded by a light transmittable jacket segment 36, forming a part of the outer liner 34, which defines an imaging compartment 38. The term "light transmittable" may include jackets made of materials that are transparent and/or translucent. The light transmittable jacket segment 36 may extend from the distal face 32 for a certain length. In another example, a light transmittable jacket surrounds a significant portion of the endoscope 10, that is, the light transmittable jacket surrounds the distal tip 22 and the intermediate region 30. Alternatively, the intermediate region 30 may include, in addition to or instead of a light transmittable jacket, an intermediate jacket (shown in **FIG. 9**) that forms another aspect of the outer liner 34. In one example, the intermediate jacket may be sized at least as long as the intermediate region and may fit over the light transmittable jacket that is extended along the body. In other examples, the light transmittable jacket segment and the intermediate jacket may be separate jackets that are coupled or integrally formed. One characteristic of the intermediate jacket may be that it reflects or directs the light beam energy toward the distal face.

The light transmittable jacket segment 36 or the intermediate jacket may be a single layer of heat shrinkable, light transmittable material. Heat shrinkable material is suitable for use in forming medical devices, such as but not limited to, polyolefin, fluoropolymer (such as fluorinated ethylene propylene (FEP), polytertrafluourethyline (PTFE) or Kynar), polyvinyl chloride (PVC), neoprene, silicone elastomer or Viton. Those skilled in the art will appreciate that various alternative compositions for the heat shrinkable jackets would also be suitable for use in forming this sheath.

The outer liner 34 may include a multi-layered structure. For example, the outer liner 34 may include an inner layer (not shown) and at least one reinforcing member (not shown), such as, for example, a braid and/or a coil. In one example, the inner jacket may comprise of a heat formable polyamide material, such as nylon, or a polyether block amide (PEBA). This heat formable material melts upon heating, such that portions flow between the respective filaments or turns of the braid or the coil (if present) and the imaging and light conductors, and bond to the inner surface of the outer line. The outer liner may be lubricous, for example comprising a polytertrafluourethyline (PTFE), or alternatively, an outer lubricious or hydrophilic coating, such as AQ® hydrophilic coating, may be applied to the outer line to allow for easier insertion within the body vessel or cavity and for allowing other devices to track over it. As may be appreciated by those of ordinary skill in the art, the braid and/or coil may be made wires and/or filaments of medical grade metal or metal alloy. Non-limiting examples of such materials include stainless steel, and shape memory alloys such as nitinol, a nickel-titanium alloy.

The endoscope body 12 may be formed to have any length required to fulfill its intended purposes. In most cases, the body may have a length between about 40 and 125 cm, and most generally, between about 70 and 100 cm. For an exemplary body 12 of 70-100 cm length, the distal tip 22 and a portion of the intermediate region 30, for example the distal 30-60 cm, may be covered with the lubricious or hydrophilic coating. In addition, if desired, the outer liner 34 may comprise two or more discrete longitudinal segments of differing durometer. Making the portions of the distal tip 22 and a distal portion of the intermediate region 30 of the outer liner, including the light transmittable jacket segment, from a lower durometer material than that from which the proximal portion is made may yield a body whose distal portion is more flexible than the proximal portion.

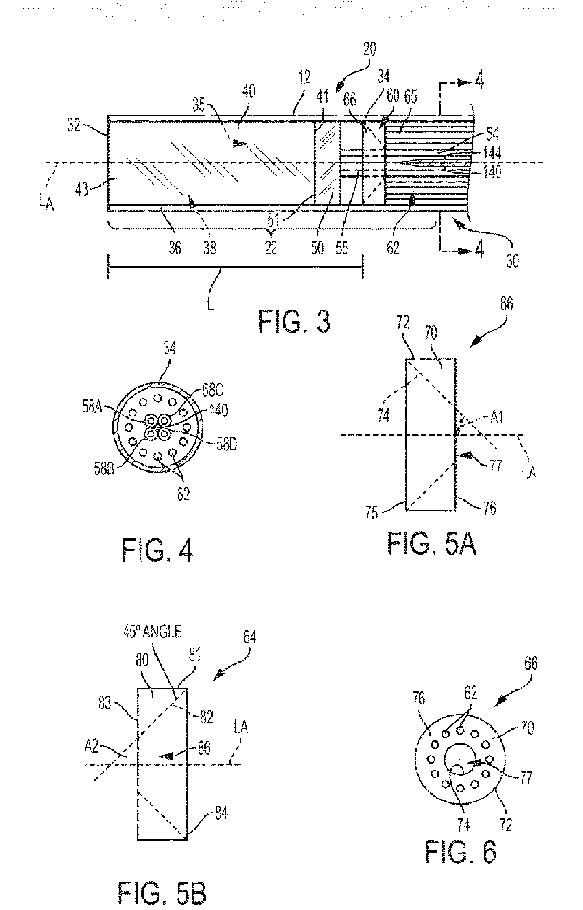

**FIG. 3** depicts the imaging system 20 in greater detail. The lens 40 and the image device 50 are arranged within the imaging compartment 38. The lens 40 is shown disposed distal to the image device 50. The lens 40 may be oriented to place its proximal lens surface 41 facing proximally a distal side 51 of the image device 50 and its distal lens surface 43, opposite the proximal lens surface 41, to coincide with and be flush with the distal face 32 of the distal tip 22, which may allow for easier cleaning, wiping or de-fogging the lens surface. The distal lens surface 43 may also be inset from the distal face 32 by a few millimeters. The location of the image device 50 proximal to the lens 40 allows the image device 50 to captures images of the view outside the distal face 32 via the lens 40. The lens 40 may be, for example, a GRIN lens, or group of lens (glass, plastic, etc.) combined to define the lens 40. The angle of view of the lens may be selected to provide adequate viewing, such as, for example, about 95 degrees to 120 degrees. In one example, the lens 40 may be available in accordance with the designs of the products manufactured by Precision Optics Corporation.

The image device 50 is configured to capture images of objects external to the endoscope 10, for example, the inner wall of the body vessel. To this end, the image device 50 may be oriented so that its imaging surface faces in the distal direction. Although not shown, a gap may exist between the proximal lens surface 41 and the distal side 51 of the image device 50. The image device 50 may comprise a solid state imaging device, such as for example, but not limited to, a complementary metal-oxide semiconductor (CMOS) camera or charge coupled device (CCD) camera. The image device 50 is configured to image the illumination reflected by the external object in response to the light energy beam of the illumination system 60, wherein the illumination is directed to pass through or refract from the lens 40 to the image device 50. The image device 50 may include a plurality of individual pixels arranged in a two-dimensional array. In one example, the image device 50 is a CMOS camera formed in accordance with the designs of the products manufactured by OmniVision Technologies, Inc., such as, its products OV6946, OV6922, or OV6930, to name a few.

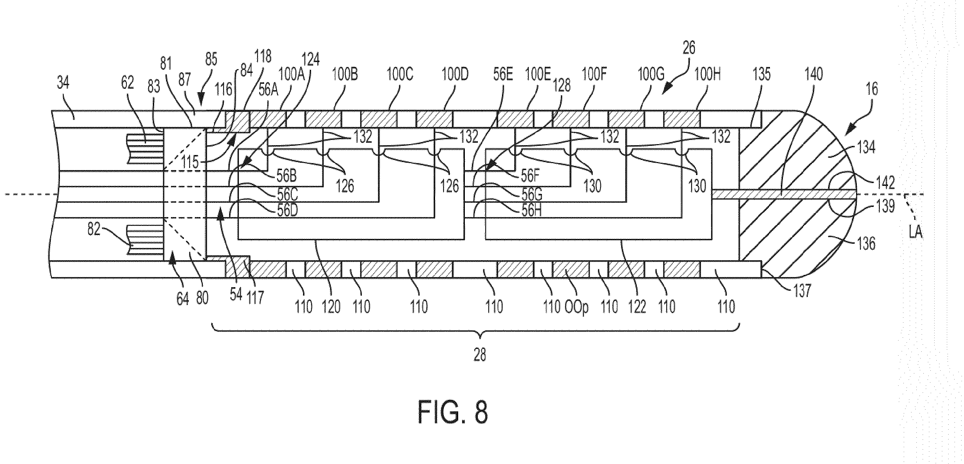

In **FIG. 3**, a plurality of image conductors 54 extend from the image device 50 to transmit driving power signals for powering the image device, to communicate image signals (optical, digital and/or analog) associated with captured images converted into electrical signals from the image device. In some instance, at least a portion of the image conductors may include fiber optic cables, in addition to the electrical conductors. Distal ends 55 of the image conductors 54 are electrically coupled to a proximal side 52 of the image device 50. A proximal end of the image conductors (shown as proximal ends 56A, 56B, 56C, 56D, 56E, 56F, 56G, 56H in **FIG. 8**), opposite to its distal ends 55, is extended within the body and oriented as will be described. For instance, the proximal ends 56A, 56B, 56C, 56D, 56E, 56F, 56G, 56H are shown in **FIG. 8** electrically coupled to a corresponding plurality of ring conductors 90A, 90B, 90C, 90D, 90E, 90F, 90G, 90H, and eventually communicated to a processor (not shown). The number of image conductors 54 will depend on the number of outputs of the image device 50. In one example shown in **FIG. 4**, the image conductors 54 comprise four coaxial cables 58A, 58B, 58C, 58D each having two conductors, resulting in a total of eight conductors.

**FIG. 3** depicts the illumination system 60 including a plurality of light conductors 62 further arranged at the distal tip 22 within the imaging compartment 38 for the imaging system 20. The light conductors 62 may be any optical fiber element suitable for transmitting light from a light source (as will be described) for emitting the light energy beam to eventually reach outside the distal face 32. The light conductors 62 may comprise any number or bundles of fiber optics and may be arranged in any configuration determined to be useful for illuminating a given procedure. The light transmittable jacket segment 36 may be used as a light conduit to illuminate beyond the distal tip 22. The termination of distal end 65 of the light conductors 62 may be at a location proximal to the image device 50 or at a longitudinal length L from the distal face 32, with the length L being defined by at least the longitudinal lengths of the lens 40 and the image device 50. This configuration may facilitate a much lower profile design by removing additional components in this region and allowing the cross-sectional area of the lens 40 and/or the image device 50, along with the wall thicknesses of any liners, to define the bounds of the cross-sectional area of the body 12.

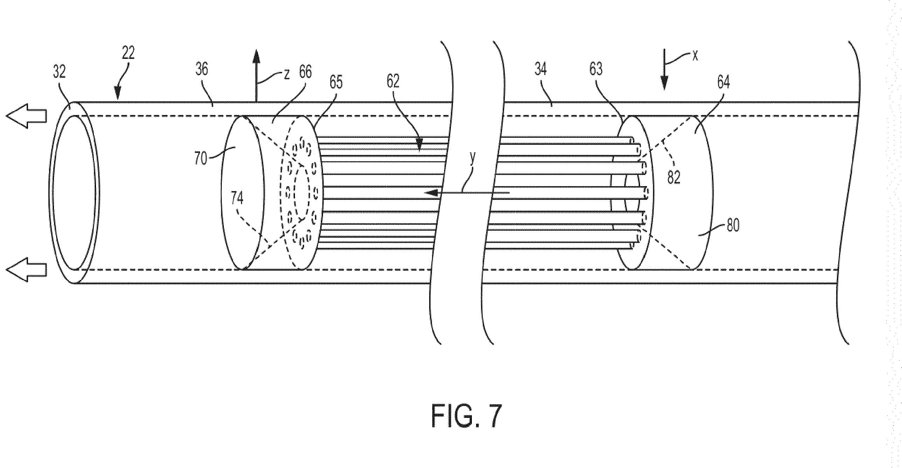

In one example, the illumination system 60 includes a proximal refractor 64, as shown in **FIG. 8**, and a distal refractor 66, as shown in **FIG. 3****.** The proximal refractor 64 is coupled to proximal ends 63 of the light conductors 62 and the distal refractor 66 is coupled to distal ends 65 of the light conductors 62. **FIG. 7** depicts this aspect of the illumination system. Light energy beam communicated radially to the proximal refractor 64 from the light source is directed longitudinally to the distal refractor 66 via the light conductors 62 that are longitudinally disposed. The distal refractor 66 is shown placed within the imaging compartment 38 proximal to the image device 50. The distal refractor 66 reflects light radially to the light transmittable jacket segment 36 disposed about the distal tip. The proximal refractor 64 is shown placed proximate the transition between the proximal end of the intermediate region 30 and the beginning of the proximal region 28, proximal to the distal refractor.

The distal refractor 66 may be configured as a right angle prism. **FIG. 5A** shows one example of the distal refractor 66. The distal refractor 66 forms an annular body 1 extending between a radially outward edge 72 and a radially inward edge 74 and having a distal axial end 75 and a proximal axial end 76. The radially inward edge 74 defines an aperture 77 of the distal refractor 66. The radially inward edge 74 may be angled at an angle A1 relative to the longitudinal axis LA, for example, by about 45 degrees, such that the aperture 77 becomes increasingly larger or tapers outwardly distally along the longitudinal axis LA. The proximal axial end 76 may be planar. The distal ends 65 of the light conductors 62 may be bonded or fixed to the proximal axial end 76 to allow for light energy transmission between the components. **FIG. 6** depicts the end view of the proximal axial end of the distal refractor 66 and an example arrangement of the light conductors radially arranged about the aperture 77.

The proximal refractor 64 may be configured as a right angle prism. **FIG. 5B** depicts one example of the proximal refractor 64. The proximal refractor 64 may form an annular body 80 extending between a radially outward edge 81 and a radially inward edge 82 and having a distal axial end 83 and a proximal axial end 84. With additional reference to **FIG. 8**, the radially outward edge 81 and body 80 of the proximal refractor 64 may define an annular window segment 85 along the serial conductor through which, in addition to another light transmittable jacket segment 87 of the outer liner 34, external light enters into body 80 and is transmitted in the distal direction through the light conductors 62. The radially inward edge 82 defines an aperture 86 of the proximal refractor 64. The radially inward edge 82 may be angled at an angle A2 relative to the longitudinal axis LA, for example, by about 45 degrees, such that the aperture 86 becomes increasingly smaller or tapers inwardly in the distal direction along the longitudinal axis LA. The distal axial end 83 may be planar. The proximal ends 63 of the light conductors 62 may be bonded or fixed to the distal axial end 83 to allow for light energy transmission between the components. The end view of the distal axial end 83 of the proximal refractor 64 would be similarly arranged as shown in **FIG. 6**

The shape of the distal and proximal refractors 66, 64 may defined by machining or with additive manufacturing of optical glass materials, such as but not limited to, fused silica, BK7, quartz, sapphire, and crown glass. Other prism geometries may be possible. The respective radially inward edges 82, 74 may function as a reflecting surface of distal and proximal refractors. The exterior surface of the radially inward edges 82, 74 and/or the proximal axial end 84 of the proximal refractor 64 and/or the distal axial end 75 of the distal refractor 66 may include a metalized material such as aluminum or a reflective coating such as aluminum coating. The distal axial end 83 of the proximal refractor 64 and/or the proximal axial end 76 of the distal refractor 66 and/or the exterior surface of the respective radially outward edges 72, 81 may remain uncoated. In another example, the light transmittable jacket segment 36 surrounding the distal tip 22 may include a metalized layer material such as aluminum or a reflective coating such as aluminum coating to direct the light energy to the distal face.

**FIG. 7** depicts a subassembly of the illumination system 60, including the proximal and distal refractors 64, 66 and the light conductors 62 extending therebetween. **FIG. 7** also illustrates the path of light communication. Light energy is emitted by a light source positioned external to the endoscope (as will be described) and is directed radially inward (arrow X) from one or more circumferential locations. The light energy beam is refracted longitudinally (about 90 degrees) in the distal direction (arrow Y) by the inclined radially inward edge 82 through the proximal refractor body 80. Light energy received longitudinally from the light conductors 62 is then refracted radially outward (about 90 degrees) by the inclined radially inward edge through the distal refractor body 70 (shown by arrow Z) to the light transmittable jacket segment 36. The light illuminates the light transmittable jacket segment 36 (up to its full circumference and its longitudinal length) to provide annular illumination of the distal tip and illumination distally beyond the distal face.

Turning to **FIG. 8**, the serial connector 26 is shown including a plurality of ring conductors 100A, 100B, 100C, 100D, 100E, 100F, 100G, 100H (collectively ring conductors 100) separated by annular insulators 110 axially disposed relative to one another along the proximal region 28. The proximal refractor 64 is further axially disposed relative to the ring conductors 100 and the annular insulators 110. A base transition insulator 115 may be positioned between the proximal refractor 64 and the first distalmost ring conductor 100A. The base transition insulator 115 may be a tubular body having a reduced diameter portion 116 and a step 117 to a larger diameter portion 118. The reduced diameter portion 116 extends distally from the larger diameter portion 118 and may be sized to fit within the proximal end of the outer liner 34. The larger diameter portion 118 may be sized to be approximately the same size of the ring conductors 100. The thickness of the step 117 corresponds with the thickness of the sidewall of the outer liner 34.

The ring conductors 100 a cylindrical tubular body configured to conduct electrical energy between the exterior surface and the interior surface. The material of the ring conductor may include but not limited to copper, aluminum, silver and other conductive materials. The ring conductor is sized to fit over the electrical circuit boards or the proximal end of the image conductors. A soldering conductor material can be applied to the ring conductor at the termination of the electric circuit board in order to electrically couple the ring conductor to the respective wire or termination lead.

The number of ring conductors 100 coincides with the total number of image conductors 54 extending from the image device 50. Any number of ring conductors 100 may be provided. Eight ring conductors 100A, 100B, 100C, 100D, 100E, 100F, 100G, 100H are shown in **FIG. 8**, while some endoscopes may have a few as two or three conductors. The image conductors 54 are shown extending longitudinally within the endoscope body 12 from the image device 50.

The proximal region 28 in **FIG. 8** defines a housing about a cavity 104 where one or more electric circuit boards may be housed. A first electric circuit board 120 and a second electric circuit board 122. The first electric circuit board 120 may include input termination leads 124 and output termination leads 126. The second electric circuit board 122 may include input terminations 128 and output termination leads 130. Proximal ends 56A, 56B, 56C, 56D of the image conductors 54 may be electrically coupled to corresponding individual input termination leads 124, and the proximal ends 56E, 56F, 56G, 56H of the image conductors 54 may be electrically coupled to corresponding individual input termination leads 128. Output termination leads 126 and 130 may be aligned with corresponding ring conductors 100A, 100B, 100C, 100D, 100E, 100F, 100G, 100H and conductor bridges 132 may be provided to electrically couple the ring conductors and the corresponding output termination. To this end, the proximal ends 56A, 56B, 56C, 56D, 56E, 56F, 56G, 56H of the image conductors 54 may be electrically coupled to corresponding ring conductors 100A, 100B, 100C, 100D, 100E, 100F, 100G, 100H.

The ring conductors 100 may be formed as a tubular body from a single piece or multiple pieces. The material of the ring conductor may include but not limited to copper, aluminum, silver and other conductive materials. A slot or opening may be formed in a sidewall of the tubular body. The opening provides access to the output terminations of the electric circuit boards to allow for conductor bridges to be added, for example, in the form of soldering conductor material applied between the ring conductors and the output terminations.

The annular insulators 110 provide insulation or prevent electrical current from passing between adjacent ring conductors 100. The annular insulators may be formed from a ring of insulating material or formed from pieces of insulating material that eventually forms the ring shape. Alternatively, the annular insulator 110 and/or the base transition insulator 115 may be formed from plastic or other material that is coated with an insulating material. Examples of insulating material include but not limited to PVC, glass, rigid laminate, resin, Teflon, and rubber. The annular insulators 110 are sized to fit over the electrical circuit boards or the proximal end of the image conductors.

A proximal plug 134 of the endoscope body 12 is shown as hemi-spherical for atraumatic purposes, although could be planar. The proximal plug 134 may provide radial strength to the proximal region 28. The proximal plug 134 includes a reduced neck portion 135 relative to a head portion 136 for insertion into the cavity 104. The transition between the neck portion 135 and the head portion 136 provides a flange 137 for capturing the ring conductors 100 and the insulators 110 in a secured fashion. The proximal plug 134 can be made from a variety of biocompatible plastics or metals. A polymer sleeve (not shown) may be provided along the interior walls of the ring conductors 100 and the annular insulators 110 and extended between the base transition insulator 115 and the proximal plug 134 to further support and increase integrity of the serial connector 26. The sleeve may be perforated to permit electrical communication between the ring conductors and the image conductors as described herein.

A safety wire 140 may be provided with in the endoscope body 12 to improve the tensile strength and other properties of the endoscope 10. The safety wire 140 is shown extending from and attached to a center lumen 139 of the proximal plug 134 with an adhesive or otherwise bonded. The safety wire 140 may be elongated member made of a Nitinol material, although it can be made from many other materials. The safety wire 140 may be made of a flexible, elastic or bendable material that is flexible enough to traverse the vessels or arteries. The safety wire 140 may be made of other materials that have properties similar to a thin spring temper stainless steel and Nitinol, such as the properties of being kink resistant, being able to withstand sterilization (heat and moisture) and being non-toxic, etc. The safety wire is extended longitudinally from its proximal end 142 in the distal direction through the intermediate of the body 12. A distal end 144 of the safety wire 140 may terminate along the intermediate region 30 of the body. For example, the distal end 144 may terminate a few millimeters from the image device and/or the distal refractor 66.

**FIGS. 9-11** depict another example of the endoscope (now referred to endoscope 150) having many of the components described with respect to the endoscope 10, and accordingly such components having common reference numerals. The endoscope 150 is shown including different embodiment of the illumination system, now referred to as illumination system 160.

In **FIG. 9**, the distal ends 165 of the light conductors 162 are in optical communication with the light transmittable jacket segment or, as shown, a distal annular ring 166. The distal ends 165 of the light conductors 162 are angled radially outward and terminating at a sidewall of the distal annular ring 166. **FIG. 10** depicts the light conductors 162 in partial annular zones 180, 182 or as needed for the desired illumination. The distal tip 22 is shown surrounded by the light transmittable jacket segment 36 and the intermediate jacket 183 that forms another aspect of the outer liner 34. The intermediate jacket 183 may reflect or direct the light beam energy toward the distal face.

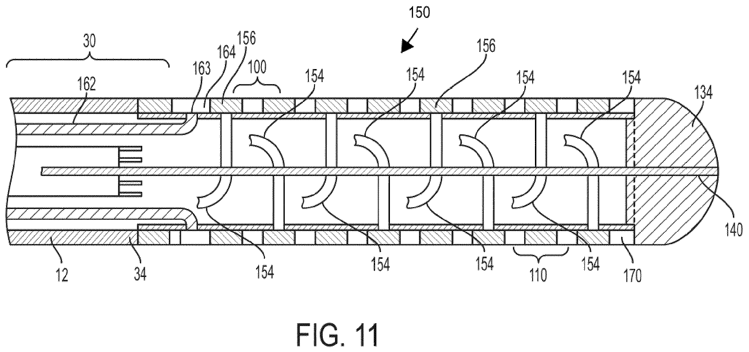

In **FIG. 11**, the proximal ends 163 of the light conductors 162 are in optical communication with another light transmittable jacket segment or, as shown, a proximal annular ring 164. The proximal ends 163 of the light conductors 162 may be angled radially outward and terminating at a sidewall of the proximal annular ring 164. The proximal annular ring 164 is axially disposed relative to the ring conductors 100 and the insulators 110. In the example shown, the proximal annular ring 164 may be disposed between the distalmost ring conductor 100 and the intermediate region 30 of the body 12. The distal and proximal annular rings 166, 164 may be a single ring structure or may be formed from several segments of optical glass or plastic material circumferentially disposed relative to one another in an annular arrangement. In some examples, the proximal ends 163 and/or distal ends 165 may be beveled to form a planar surface for bonding or attachment to the respective rings 164, 166.

**FIG. 11** also depicts another embodiment of the serial connector without the use of electric circuit boards. Instead, the proximal ends 156 of the image conductors 154 extend radially outward from the intermediate of the endoscope body 12 to a corresponding ring conductor 100. For illustrative purposes, portions of the image conductors 154 are removed in **FIG. 11****.** The proximal ends may be extended through the slot of the ring conductor and may be soldered or other in electrical communication with the corresponding ring conductor. Any extensions after soldering can be trimmed and filed for a smoother surface. A proximal polymer sleeve 170 is shown placed over the image conductors 154 and along the interior walls of the ring conductors 100 and the annular insulators 110 for further supporting and increasing the integrity of the serial connector 26. The sleeve 170 may be extended from the proximal plug 134 to underneath the proximal end of the outer liner 34 in an overlapping relationship. The sleeve 170 may be perforated to permit electrical communication between the ring conductors 100 and the image conductors 154 as described herein.

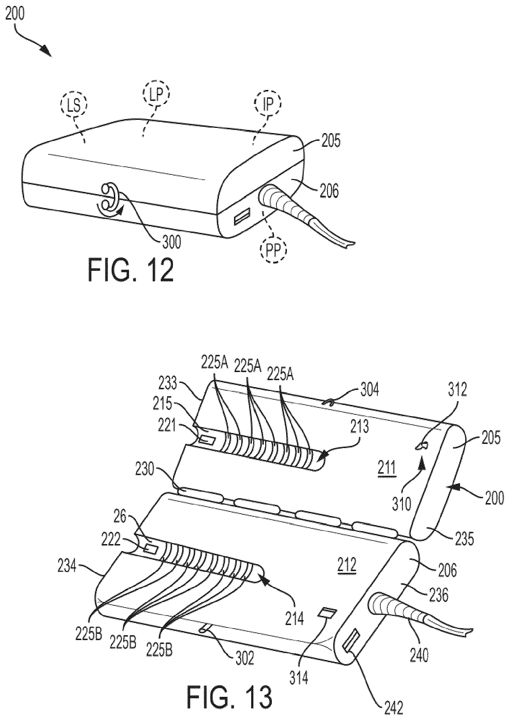

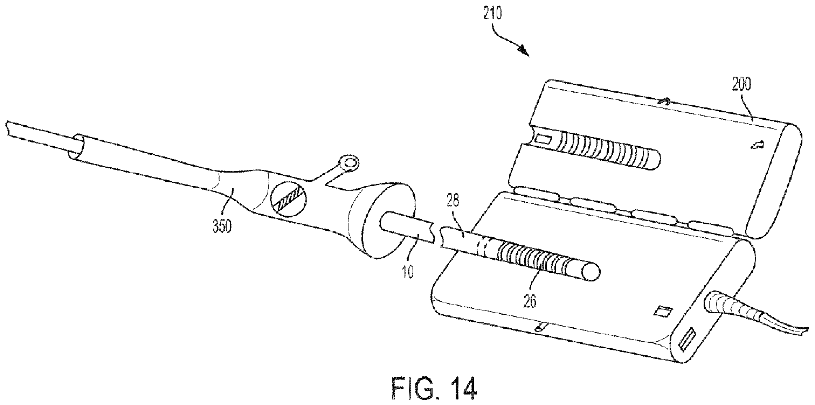

With reference to **FIGS. 12-13**, the endoscope 10 or 150 may be combined with an electrical-light connector 200. The description below will focus on the endoscope 10, and it can be appreciated that the description would be applicable to the endoscope 150. **FIG. 14** shows the electrical-light connector 200 may be removably attached to the proximal region 28 including the serial connector 26 of the endoscope 10 to define a vision system 210. The electrical-light connector 200 includes a light source LS, as well as one or more controllers for image processing IP, light processing LP, and/or power processing PP shown contained within a housing (shown as first and second housings 205, 206) of the electrical-light connector 200. The housings and other components of the electrical-light connector 200 can be made of medical grade plastics or metals, as can be appreciated by one of ordinary skill in the art.

The housings 205, 206 include interfacing surfaces 211, 212, respectively, that define respective longitudinal arcuate grooves 213, 214. When the interfacing surfaces 211, 212 are placed in a confronting relationship such as when the electrical-light connector 200 is in the closed position shown in **FIG. 12**, the grooves 213, 214 together define a channel sized and shape to receive the serial connector 26 of the endoscope 10.

The surfaces 215, 216 that define the respective grooves 213, 214 each includes a light window segment 221, 222 that is in optical communication with the light source contained within the housings 205, 206. For example, an aperture may be formed into the groove defining surfaces 215, 216 (shown at the base of the groove), where the aperture defines the frame of the light window segments 221, 222. The light window segments 221, 222 may be left without a protective lens cover. In some examples, a protective lens coupled to and supported by the frame. The lens may be coupled in a manner where a seal is formed in order to inhibit debris or fluids from entering into the housing cavities. The protective lens may also be configured to amplify or intensify the light energy beam generated by the light source. The light source may comprise one or more light sources, including a light-emitting diode (LED), laser, or other suitable light emitting devices. The light source may be suitably positioned or otherwise configured to emit and direct a light energy beam through the respective light window segments 221, 222.

The groove defining surfaces 215, 216 each includes a series of curved conductors 225A, 225B that are in electrical communication with the one or more controllers for image processing and power processing. The curved conductors 225A, 225B are axially spaced relative to one another in a similar manner as the ring conductors 100, with spacing in between the curved conductors coinciding with the spacing formed by the annular insulators. In one example, this spacing between adjacent curved connectors may include a coating or piece of insulating material. In one example, the curved conductors 225A, 225B may be shaped and sized to occupy a segment of the groove defining surfaces 215, 216. To this end, when the electrical-light connector 200 is in the closed position, the curved connectors 225A, 225B define pairs of conductor in an annular arrangement that will be in surrounding contact and in coaxial relationship with the corresponding ring conductors 100. Alternatively, the curved conductors need not occupy the entire circumference or even be located in both grooves, so long as a contacting and communication relationship is formed between the ring conductors and the curved conductors. In another example, the curved conductors may be inset into the groove defining surfaces 215, 216 in order to present a generally smooth surface. Holes (not shown) may be formed underneath the curved conductors where electrical conductive material (wires and/or solder) may be extended between the curved conductors and the respective controller boards. Curved conductors may be made of any one of the conductor materials of the ring conductors.

The housings 205, 206 are shown pivotably attached together along a hinge 230. One or both of the housings 205, 206 include a cavity defined therein that houses the light source and the one or more controllers for image processing, light processing, and/or power processing, as can be appreciated by one of ordinary skill in the art. The housings 205, 206 include a receiving end 233, 234, respectively, and a non-receiving end 235, 236, respectively. The endoscope is inserted at and extends from the receiving ends 233, 234. To this end, the groove defining surfaces 215, 216 extend all the way through the receiving ends 233, 234 from an intermediate portion of the interfacing surfaces 211, 212, respectively. A power cable is shown coupled to the non-receiving end 236 of the housing 206. The power cable 240 provides power from an external source to the electrical-light connector 200 and ultimately to the light source and the image device of the endoscope 10 via the transmission of power via the various conductors. A communication port 242 (shown as a USB port) is also shown coupled to the non-receiving end 236 of the housing 206. An external display monitor(s) may be coupled to the communication port 242 via a communication conductor. The communication port 242 communicates video image signals from the one or more controllers for image processing that has processed such signals from the image device of the endoscope via the various conductors. As can be appreciated by one of ordinary skill in the art, the housing may be formed from a single piece having a channel formed therein and the walls defining the channel having the arrangement of the light window segments and curved conductors as shown in the figures.

The one or more controllers for image processing, light processing, and/or power processing (not shown) may be placed in the electrical-light connector 200. The light source emits a light energy beam or beams radially through the respective light window segments 221, 222 to the annular window segment defined by the proximal refractor 64, and ultimately to the distal tip 22 via the distal refractor 66 and the light conductors 62. The light source may be altered (intensity, gating, filtering) and/or switched on and off by being in communication with a controller for light processing. The light energy beams emitted outside the distal tip 22 is directed at an object. Beams of reflected illumination transmitted via the lens 40 are recorded by the image device 50. The image device 50 may digitally record and convert the recorded image to the imaging signal that is communicated through the image conductors 54 and eventually, via the ring conductors 100 and curved conductors 225A, 225B, to the one or more controllers for image processing housed in the electrical-light connector 200. The image signals may be further filtered and separated by the one or more controllers for image processing. The one or more controllers for image processing may perform the function of processing image signals to form an image. To this end, the one or more controllers for image processing may include imaging software for processing and displaying these images on the external display monitor(s) (not shown) coupled to the communication port 242 via a communication conductor (not shown).

The electrical-light connector 200 is movable between the closed position and the open position. In the open position, the housings 205, 206 are moved away by pivoting away from one another along the hinge 230 such that the serial connector 26 of the endoscope 10 may be inserted and positioned within the groove 214. In the closed position, the housings 205, 206 are moved together by pivoting toward one another along the hinge 230 such that the serial connector 26 of the endoscope 10 is inserted and positioned within the channel defined by the grooves 213, 214.

Alternatively, the illumination system of the endoscope 10 or 150 may include a LED light source (not shown) in place of the distal and proximal refractor and light conductor system. The LED light source may be placed where the distal refractor is located within the imaging compartment 38 of the distal tip 22. The LED light source is placed so that a light-emitting surface is located proximate the light transmittable jacket segment. The LED light source may be supplied with power through power conductors that would run in a similar location as the light conductors. A proximal end of each power conductor may be coupled to the LED light source. A distal end of the power conductors may be coupled to an additional ring conductor similar to how the image conductors are attached. The electrical-light connector 200 may include another series of curved conductors in place of the light window segments 221, 222 such that one or more controller for light processing is coupled to the LED light source via the curved conductors, the ring conductors, the power conductors for controlling the LED light source.

When the interfacing surfaces 211, 212 are placed in a confronting relationship such as when the electrical-light connector 200 is in the closed position shown in **FIG. 12**, the grooves 213, 214 together define a channel sized and shape to receive the serial connector 26 of the endoscope 10. When the serial connector 26 is received by the channel defined by the grooves 213, 214, the light window segments 221, 222 and the proximal refractor 64 are in alignment to allow for optical communication from the light source to the distal tip 22 via the proximal and distal refractors 64, 66 and the light conductors 62. The curved conductors 225A, 225B are in alignment with corresponding ring conductors 100 of the serial connector 26 to allow for electrical communication from the electrical-light connector 200 to the image device 50 via the image conductors 54.

The electrical-light connector 200 may include other features. For example, the electrical-light connector 200 may include a locking device 300. The locking device 300 is configured to maintain the housings 205, 206 in the closed position around the serial connector 26 of the endoscope 10. Various locking devices can be used. In one example, the locking device includes a post 302 coupled to the housing 206 and a pivot latch 304 pivotably coupled to the housing 205. The pivot latch 304 swings about one end and include a hooked end to capture the post 302 in the locking configuration. The pivot latch 304 can be decoupled from the post and pivoted away to the unlocked configuration. Alternatively, the post and the pivot latch can be coupled to the other housing.

The electrical-light connector 200 may include a switch 310 to turn off and turn on the electrical-light connector 200. The switch 310 may be a manual rocker switch or push button. In one example, the switch 310 comprises an auto configuration as shown in **FIG. 13****.** Here, the switch 310 includes a switch post 312 coupled to the interfacing surface 211 and a switch aperture 314 formed in the interfacing surface 212. The switch aperture 314 is configured to receive the switch post 312 when the electrical-light connector 200 is in the closed position. The switch post 312 may make contact with a component within the housing to allow for power and light between the electrical-light connector 200 and the endoscope 10. When the switch post 312 is removed from contact with such component within the housing, power and light between the electrical-light connector 200 and the endoscope 10 is disconnected.

The endoscope may be employed in body vessels and other body organs and structures for imaging such, such as the esophagus, the stomach, the colon, the uterus, saphenous vein grafts, heart valves, and other body cavities, lumens, channels, and canals. The following description is merely one example of an application and will focus on imaging the ureters and kidneys, for example, for the physician operator to obtain a diagnosis following kidney stone symptoms.

A physician may place an introducer sheath or a urinary catheter through a urethra and the bladder. The endoscope 10 or 150 and the electrical-light connector 200 in the closed position are coupled to one another. The endoscope may be introduced within the urinary catheter and advanced to a ureter into a kidney, using the illumination system and imaging system for navigation. In the event more steerability is desired, the endoscope is immobilized and the electrical-light connector 200 is moved to the open position and the endoscope 10 or 150 becomes decoupled from the electrical-light connector 200. The sheath or catheter may then be removed in the proximal direction from over the proximal end of the endoscope. A steerable catheter 350 having a longitudinal lumen in turn may be advanced in the distal direction over the proximal end of the endoscope, as shown for example in **FIG. 2****.** The electrical-light connector 200 is again oriented over the serial connector of the endoscope and is moved to the closed position, as shown in **FIG. 14****.** The endoscope 10 or 150 becomes energized from the coupling with the electrical-light connector 200. Once the position is re-identified via the endoscope, navigation may resume.

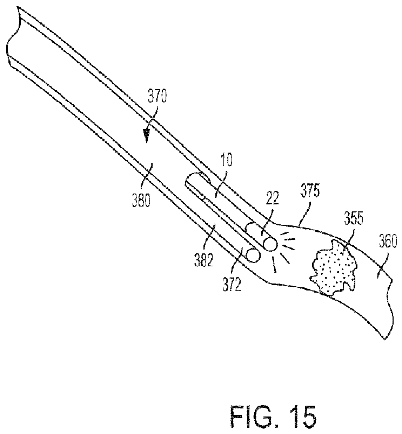

With reference to **FIG. 15**, the distal tip 22 of the endoscope is advanced with the overlying steerable catheter, providing images for the physician to investigate or find a treatment site 355 that requires therapy within the body vessel 360. Once visual confirmation has been made of the treatment site 355 and condition, the endoscope 10 may be immobilized and the electrical-light connector 200 is moved to the open position to decouple from the endoscope 10 or 150. The steerable catheter may then be removed in the proximal direction from over the proximal end of the endoscope 10. A treatment device 370, such as lithotripsy, laser or basket devices, may be advanced in the distal direction over the proximal end of the endoscope 10. The electrical-light connector 200 is again oriented over the serial connector of the endoscope 10 and is moved to the closed position. The endoscope 10 becomes energized from being coupled with the electrical-light connector 200, as referenced by the light beam 375.

Once the position is re-identified via the endoscope 10, the treatment device 370 may be advanced over the endoscope 10 such that a distal end 372 of the treatment device 370 may be advance further than the distal tip 22 of the endoscope to perform treatment. The configuration of the treatment device 370 happens to be a rapid exchange configuration. Here, a proximal sheath housing 380 of the treatment device 370 is sized to fit over the body of the endoscope 10, and a distal tubular portion 382 extends beyond the distal end of the proximal sheath housing 380. The obstruction or stone at the treatment site 355 may be captured by the treatment instrument residing within the distal tubular portion 382 and the treatment catheter 370 may be withdrawn proximally over the endoscope with the captured stone. One of ordinary skill in the art would understand that the treatment catheter and the endoscope may be then removed.

The endoscope 10 or 150 may be constructed in the following manner. The light conductors may be prepared by trimming to length. The distal and proximal refractors may each be separated or manufactured into halves. The proximal ends of the wires may be attached or bonded to the proximal refractor halves. The distal ends of the wires may be attached or bonded to the distal refractor halves. The image conductors may be a part of the image device and may be ordered with the correct length. In this example, the image conductors may be preassembled to dual 4-pin circuit boards. The distal refractor halves may be spaced appropriately from the image device and placed over the image conductors and attached or bonded to one another. The proximal refractor halves may be spaced appropriately from the distal refractor assembly to extend the light conductors and placed over the image conductors and attached or bonded to one another. The distal and proximal refractors may be temporarily bonded to the image conductors in order to fix their relative position. The bundle now may be twisted in one direction to reduce bunching during deflection and to strengthen the core of the body.

A light transmittable heat shrink jacket is placed and pulled over the entire bundle. Heat shrink jackets for use in forming medical devices are well known in the art, with fluorinated ethylene propylene (FEP) being an exemplary composition for use herein. The light transmittable heat shrink jacket may be pulled beyond the image device to further define the imaging compartment for the lens and may be sized appropriately to extend beyond the proximal refractor. The lens is then placed into the tip of the light transmittable heat shrink jacket and spaced appropriately from the image device. An ultraviolent adhesive may be used on the lens to bond it to the light transmittable heat shrink jacket. Heat sufficient to activate the light transmittable heat shrink tube may be applied to the distal end to immobilize the lens, the image device and the distal refractor. The heat shrink jacket and contents are heated (typically at about 385° F (196° C) when FEP is used as the heat shrink jacket. The intermediate region of the light transmittable heat shrink tube may be heated gradually from the distal end and in the proximal direction to ensure that no air is entrapped, stopping short of the proximal end. The proximal refractor may be immobilized during the heat shrinking.

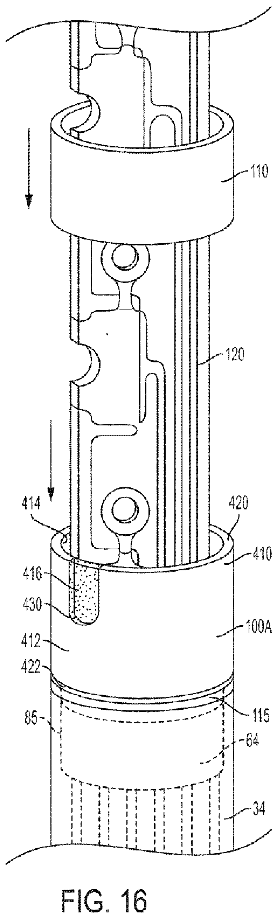

With reference to **FIG. 16**, the base transition insulator 115 may be placed over the electric circuit board 120 and slid down to the proximal end of the light transmittable heat shrink tube forming the outer liner 34 so that the base transition insulator 115 is placed adjacent the proximal refractor 64. The reduced diameter portion of the base transition insulator 115 is placed within the proximal end of the light transmittable heat shrink tube. Heat sufficient to activate the light transmittable heat shrink tube may be applied to the proximal end to bring it down around the reduced diameter portion of the base transition insulator 115. The rest of the light transmittable heat shrink tube may be trimmed.

The ring conductors 100 are next introduced to the assembly. **FIG. 16** depicts one example of the ring conductors (referred to as the ring conductor 100A, having a tubular body 410 with an exterior surface 412 and a lumen defining surface 414. A longitudinal slot 416 may be formed in a sidewall of the tubular body 410 extending from one of its proximal end 420 or distal end 422 (shown extending from the proximal end 420) and terminating along the intermediate portion of the body between the proximal and distal ends 420, 422. The longitudinal slot 416 is sized and shaped to receive wire extending from the image conductors from within the lumen of the ring conductor or otherwise access to the electric circuit board 120. A soldering conductor material 430 can be applied to the wire end of the image conductor or termination lead of the electric circuit board 120 in order to electrically couple the ring conductor to the respective image conductor or termination lead.

The first distalmost ring conductor 100A is placed over the over the electric circuit board 120 and slid in the distal direction (shown by the arrow) into place adjacent the base transition insulator 115, and the soldered to the correct lead via the slot 416 in the ring conductor 100A. The annular insulator 110 is slid over the electric circuit board 120 in the distal direction (shown be the arrow) and placed against the ring conductor 100A. Subsequent ring conductors and insulators are placed over the electric circuit boards and the soldering of the ring conductors to the appropriate lead of the electric circuit boards. After the appropriate number of ring conductors and insulators have been attached, the proximal plug is attached to the last insulator. The safety wire may be inserted into the light transmittable heat shrink tube and through the proximal and distal refractors and adjacent to the image conductors prior to heat shrinking the tube with its proximal end extending out for coupling to the proximal plug. The safety wire being fixed by heat shrinking with the bonded proximal plug may facilitate capturing the ring conductors and the insulators in a secure manner. In another example, the proximal sleeve may be placed over the electrical circuit boards or over the image conductors.

While various embodiments of the invention have been described, the invention is not to be restricted except by the attached claims. Moreover, the advantages described herein are not necessarily the only advantages of the invention and it is not necessarily expected that every embodiment of the invention will achieve all of the advantages described.

## Claims

1. An endoscope (10), comprising:
an elongated body (12) having an outer liner (34) that defines a lumen (35) disposed about a longitudinal axis, the body having a proximal end (16) and a distal tip (22), the outer liner having at least a portion of a light transmittable jacket (36) disposed about the distal tip;
an imaging system (20) including a lens (40) and an image device (50) disposed within the lumen at the distal tip, and a plurality of image conductors (54) extending within the lumen and having a distal end coupled to the image device and a proximal end,
a plurality of light conductors (62) arranged at the distal tip, the light conductors having a distal end (65) and a proximal end (63) and extending within the lumen therebetween, the distal end of each of the light conductors in optical communication with the light transmittable jacket at a location proximal to the image device; and
a serial electrical connector (26) formed along the proximal end of the body, the serial electrical connector comprising a plurality of ring conductors (100; 100A to 100H) spaced from one another and an annular window segment (85) axially disposed relative to the ring conductors, each of the ring conductors associated with and in electrical communication with the proximal end of one of the image conductors, the annular window segment in optical communication with the proximal end of the light conductors.

2. The endoscope of claim 1, wherein the distal tip of the body has a cross-sectional area that is at least as large as the proximal end of the body.

3. The endoscope of any one of the preceding claims, wherein the body has a uniform profile from its proximal end to the distal tip.

4. The endoscope of any one of the preceding claims, wherein the distal end of each of the light conductors is angled radially outward and terminating at a wall of the light transmittable jacket.

5. The endoscope of any one of the preceding claims, wherein the annular window segment comprises a proximal refractor disposed with the lumen proximate to the ring conductors, preferably
further comprising a distal refractor disposed within the lumen proximal to the image device, wherein the proximal end of the light conductors is coupled to the proximal refractor and the distal end of the light conductors is coupled to the distal refractor and preferably
wherein each of the proximal and distal refractors is a right angle prism.

## Patentansprüche

1. Endoskop (10), umfassend:
einen länglichen Körper (12) mit einer äußeren Auskleidung (34), die ein um eine Längsachse angeordnetes Lumen (35) definiert, wobei der Körper ein proximales Ende (16) und eine distale Spitze (22) aufweist, wobei die äußere Auskleidung zumindest einen Abschnitt eines lichtdurchlässigen Mantels (36) aufweist, der um die distale Spitze angeordnet ist;
ein Bildgebungssystem (20) mit einer Linse (40) und einem Bildgebungsgerät (50), das im Lumen an der distalen Spitze angeordnet ist, und eine Vielzahl von Bildleitern (54), die sich im Lumen erstrecken und ein mit dem Bildgebungsgerät gekoppeltes distales Ende und ein proximales Ende aufweisen,
eine Vielzahl von Lichtleitern (62), die an der distalen Spitze angeordnet sind, wobei die Lichtleiter ein distales Ende (65) und ein proximales Ende (63) aufweisen und sich im Lumen dazwischen erstrecken, wobei das distale Ende jedes der Lichtleiter mit dem lichtdurchlässigen Mantel an einer Stelle proximal zum Bildgebungsgerät in optischer Verbindung steht; und
einen seriellen elektrischen Verbinder (26), der entlang des proximalen Endes des Körpers ausgebildet ist, wobei der serielle elektrische Verbinder eine Vielzahl von voneinander beabstandeten Ringleitern (100; 100A bis 100H) und ein bezüglich den Ringleitern axial angeordnetes ringförmige Fenstersegment (85) umfasst, wobei jeder der Ringleiter mit dem proximalen Ende eines der Bildleiter in Zusammenhang und in elektrischer Verbindung steht, wobei das ringförmige Fenstersegment mit dem proximalen Ende der Lichtleiter in optischer Verbindung steht.

2. Endoskop nach Anspruch 1, wobei die distale Spitze des Körpers eine Querschnittsfläche aufweist, die mindestens so groß wie das proximale Ende des Körpers ist.

3. Endoskop nach einem der vorhergehenden Ansprüche, wobei der Körper ein gleichförmiges Profil von seinem proximalen Ende zur distalen Spitze aufweist.

4. Endoskop nach einem der vorhergehenden Ansprüche, wobei das distale Ende jedes der Lichtleiter radial auswärts angewinkelt ist und an einer Wand des lichtdurchlässigen Mantels endet.

5. Endoskop nach einem der vorhergehenden Ansprüche, wobei das ringförmige Fenstersegment einen proximalen Refraktor umfasst, der mit dem Lumen neben den Ringleitern angeordnet ist, vorzugsweise
ferner umfassend einen distalen Refraktor, der im Lumen proximal zum Bildgebungsgerät angeordnet ist, wobei das proximale Ende der Lichtleiter mit dem proximalen Refraktor verbunden ist und das distale Ende der Lichtleiter mit dem distalen Refraktor verbunden ist, und vorzugsweise
wobei jeder der proximalen und distalen Refraktoren ein rechtwinkliges Prisma ist.

## Revendications

1. Endoscope (10) comprenant :
un corps allongé (12) possédant une doublure externe (34) qui délimite une lumière (35) disposée autour d'un axe longitudinal, le corps présentant une extrémité proximale (16) et une pointe distale (22), la doublure externe possédant au moins une partie d'une chemise de transmission de lumière (36) disposée autour de la pointe distale ;
un système d'imagerie (20) comprenant une lentille (40) et un dispositif d'image (50) disposé à l'intérieur de la lumière au niveau de la pointe distale, et une pluralité de conducteurs d'images (54) s'étendant à l'intérieur de la lumière et possédant une extrémité distale couplée au dispositif d'image et une extrémité proximale,
une pluralité de conducteurs de lumière (62) agencés au niveau de la pointe distale, les conducteurs de lumière ayant une extrémité distale (65) et une extrémité proximale (63) et s'étendant dans la lumière entre ceux-ci, l'extrémité distale de chacun des conducteurs de lumière étant en communication optique avec la chemise de transmission de lumière au niveau d'un emplacement proximal par rapport au dispositif d'image ; et
un connecteur électrique en série (26) formé le long de l'extrémité proximale du corps, le connecteur électrique en série comprenant une pluralité de conducteurs annulaires (100 ; 100A à 100H) espacés les uns des autres et un segment de fenêtre annulaire (85) disposé de manière axiale par rapport aux conducteurs annulaires, chacun des conducteurs annulaires étant associé à une extrémité proximale de l'un des conducteurs d'image et en communication électrique avec celle-ci, le segment de fenêtre annulaire étant en communication optique avec l'extrémité proximale des conducteurs de lumière.

2. Endoscope selon la revendication 1, dans lequel la pointe distale du corps présente une coupe transversale qui est au moins aussi grande que l'extrémité proximale du corps.

3. Endoscope selon l'une quelconque des revendications précédentes, dans lequel le corps possède un profil uniforme depuis son extrémité proximale vers la pointe distale.

4. Endoscope selon l'une quelconque des revendications précédentes, dans lequel l'extrémité distale de chacun des conducteurs de lumière est inclinée de manière radiale vers l'extérieur et se termine au niveau d'une paroi de la chemise de transmission de lumière.

5. Endoscope selon l'une quelconque des revendications précédentes, dans lequel le segment de fenêtre annulaire comprend un réfracteur proximal disposé avec la lumière à proximité des conducteurs annulaires, de préférence
comprenant en outre un réfracteur distal disposé à l'intérieur de la lumière près du dispositif d'image, dans lequel l'extrémité proximale des conducteurs de lumière est couplée au réfracteur proximal et l'extrémité distale des conducteurs de lumière est couplée au réfracteur distal et de préférence
dans lequel chacun des réfracteurs proximal et distal est un prisme à angle droit.
